# EUROPEAN PATENT SPECIFICATION

(11) **EP 0 542 837 B1**
(45) Date of publication and mention of the grant of the patent: **10.05.1995**
(21) Application number: 91914457.6
(22) Date of filing: 07.08.1991
(51) Int. Cl.: C07C 215/54, A61K 31/135

(54) **PROPYLAMINE DERIVATIVES**
PROPYLAMINEDERIVATE
DERIVES DE PROPYLAMINE

(30) Priority: 08.08.1990 GB 9017390
(43) Date of publication of application: 26.05.1993
(73) Proprietor: NORGINE LIMITED, Mid Glamorgan CF8 8SJ (GB)
(72) Inventor: HORGAN, William, J. 116-129 London Road, Oxford 0X3 9BA (GB)
(74) Representative: King, James Bertram
(86) International application number: GB9101348
(87) International publication number: WO9202488

(56) References cited:
- FR-A- 1 515 687
- GB-A- 115 410
- NL-A- 660 676
- NL-C- 133 871
- CHEMICAL ABSTRACTS, vol. 78, no.11, 19 March 1973, Columbus Ohio US, abstract no. 67072D, IRESON, J,D,:"Effects of Alverine citrate on smooth muscle" page 38; column 1;& PHARMACOLOGICAL RESEARCH COMMUNICATIONS vol. 4, no. 3, 1972, pages 191-194; & CHEMICAL SUBSTANCE INDEX, page 5429CS "Benzenep ropanamine, N-ethyl-N-(3-phenylpropyl).

## Description

Certain nitrogen containing materials, sometimes in the form of amines or quaternary bases, are known to possess antispasmodic activity.

The present invention provides a propylamine derivative (I) (hereinafter defined) which we have shown to possess significant antispasmodic properties.

The compound (I) is probably best used in one of its salt forms, e.g. its hydrochloride, as this increases water solubility and may improve its bioavailability.

According to one aspect of the invention there is provided a propylamine derivative having the general formula (I)

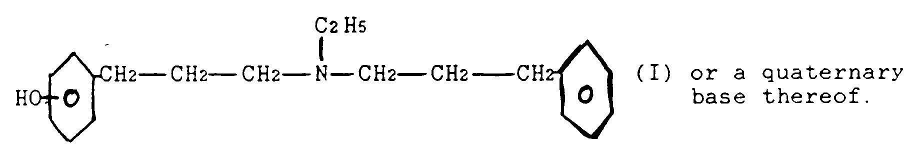

An especially useful compound within the general formula (I) is compound (II) which is N-ethyl, N-(3-phenyl propyl), 3-(4-hydroxyphenyl)-propylamine, i.e. the para hydroxy compound.

The said propylamine derivative (I) or (II) may be in the form of a pharmaceutically acceptable inorganic salt, e.g. the hydrochloride thereof, or organic salt, e.g. the citrate.

According to another aspect of the invention there is provided the use of the compound (I) or a quaternary base thereof, or a pharmaceutically acceptable salt thereof as an antispasmodic agent.

According to a further aspect of the invention there is provided a composition comprising a compound (I) or a quaternary base thereof or a pharmaceutically acceptable salt thereof and a pharmaceutically acceptable carrier therefor. Such a composition may be in the form of tablets, capsules, microspheres, liquid, semisolid or suppository dosage. Such a composition may be administered by oral, intravenous, intramuscular, topical or rectal route.

According to a still further aspect of the invention there is provided a method for the production of compound (I) comprising coupling, using a coupling agent, a compound having the formula (III) 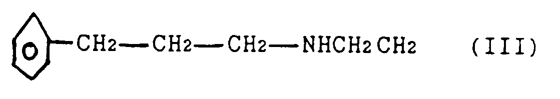
with a compound having the general formula (IV) 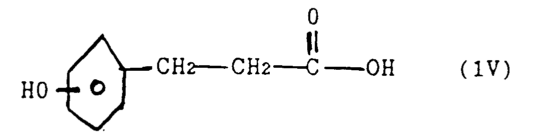
to produce a compound having the general formula (V) 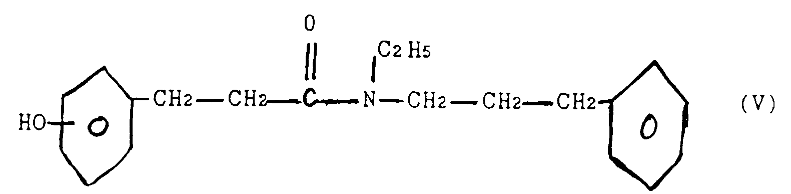
and reducing, using a reducing agent, the compound (V) to the amine having the general formula (I) as defined above.

Preferably the said coupling agent is dicyclohexyl carbodimide, preferably in methylene chloride, and preferably the said reduction is achieved by the use of lithium aluminium hydride as reducing agent, preferably in diethyl ether.

### Preparative Example

### Preparation of N-ethyl, N-(3-phenyl propyl), 3-(4-hydroxyphenyl)-propylamine (II)

The synthesis commences with 3-(phenyl) propionic acid (dihydrocinnamic acid) (a) having the formula 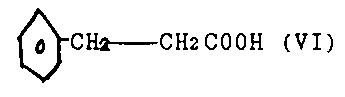
which is converted to the corresponding acid chloride having the formula 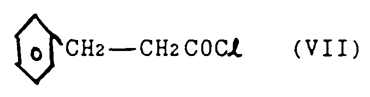
with thionyl chloride (b). Treatment of this acid chloride (VII) with a large excess of anhydrous ethylamine gives a white crystalline amide having the formula (VIII) 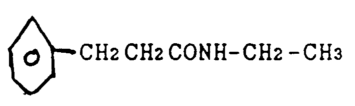
in good yield. Reduction of the said amide with lithium aluminium hydride worked well in diethyl ether (c) (but not tetrahydrofuran) to give a thick yellow brown oil which was slower on TLC (thin layer chromatography). The distinctive pair of H² protons were observed at 1.8 ppm in the proton nuclear magnetic (¹H NMR) spectrum. The product could be extracted into aqueous acid and gave a positive colour test with benzyl p-nitrophenyl formate due to the liberation of p-nitrophenol (yellow). Both of these are positive tests for the presence of an amine group.

The resultant amine which has the formula 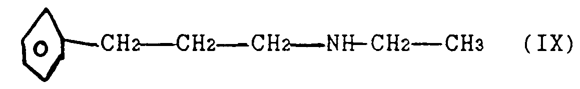
was successfully coupled with 3-(p-hydroxyphenyl) propionic acid having the formula 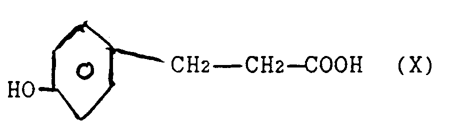
using dicyclohexyl carbodimide (DCC) in methylene chloride. The major part of the by-product, dicyclohexyl urea (DCU), was removed by filtration of the crude reaction mixture and the rest precipitated from a concentrated solution after aqueous work-up. The tertiary amide having the formula (XI) 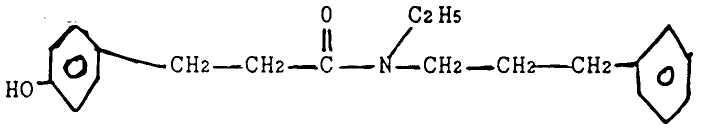
gave the characteristic AB splitting pattern of the p-hydroxyalkyl moiety in the aromatic region of the ¹H NMR spectrum.

The amide (XI) was reduced using lithium aluminium hydride in diethyl ether, where the ether to amide ratio was in the range 20:1 to 30:1, (a b c) to give the required product N-ethyl, N-(3-phenyl propyl), 3-(4-hydroxyphenyl)-propylamine (II).

### EXPERIMENTAL

### N-ethyl 3-phenyl propylamide (VIII)

Prepared according to literature procedure. (b) ¹H NMR 7.6 (5H, s, aromatics); 3.2 (2H, m, 2H²); 2.9 (2H, m 2H⁴); 2.4 (2H, m, 2H¹); 1.0 (3H, t, 7hz, 3H⁵).

### N-ethyl 3-phenylproplylamine (IX)

Lithium aluminium hydride (5.78 g, 152 mmoles) was dissolved in dry diethyl ether (250 ml) in a 3 neck 1L flask fitted with a dropping funnel, reflux condenser, stopper and magnetic stirrer bar. All exits were closed with drying towers. The solution was brought to reflux and the heat removed. The amide (VIII) (16 g, 90 mmoles) dissolved in dry diethyl ether (250 ml) was added dropwise over 30 minutes with vigorous stirring. The solution was then refluxed for 36 hours. Water (700 ml) was added cautiously; the gummy gel was filtered and the filtrate extracted with ether (5 x 350 ml). The combined organic extracts were dried over magnesium sulphate, filtered and evaporated to give a yellow oil (14.5 g, 100%).

| | |
|---|---|
| TLC Starting material | Rf 0.46 |
| Product | Rf 0.2 |

Silica gel plates, eluant 98% chloroform, 2% methanol/1H NMR (CDC1₃) 7.2 ppm (5H, m, aromatics); 2.65 (6H, m, 2H¹, 2H³, 2H⁴); 1.8 (2H, m, 2H²); 1.1 (3H, t, 7hz, 3H⁵). N-ethyl, N-(3-phenylpropyl), 3-(4-hydroxyphenyl)-propylamide (XI)

The acid (X) (50 g, 0.3 mole) and DCC (32 g, 0.16 mole) were suspended in methylene chloride (200 ml). The amine (IX) (16 g, 0.1 mole) was added dropwise from a pressure equalised dropping funnel. A mild exotherm was produced. After two hours stirring the reaction mixture was filtered and the filtrate poured into saturated sodium bicarbonate (300 ml) which was extracted with methylene chloride (5 x 200 ml). The collected extracts were dried with sodium sulphate and evaporated to give an orange oil (51.68 g). The precipitate which was filtered off was shown to consist entirely of DCU by ¹H NMR. The oil was dissolved in hot benzene, additional DCU precipitated and was filtered off (yield 30 g, 93%). ¹H NMR (CDC1₃) 7.5-6.9 ppm (10H, m, aromatics, OH); 3.5-2.2 (10H, m, 2H¹, 2H², 2H⁴, 2H⁶, 2H⁷); 2-1.5 (2H, m, 2H⁵); 1.1 (3H, t, 7hz, 3H⁸).

### N-ethyl, N-(3-phenylpropyl), 3-(4-hydroxyphenyl)-propylamine (II).

Lithium aluminium hydride (20 g, 52 mmoles) was dissolved in dry diethyl ether (1L, CaH₂ dried) in a 10L three neck flask fitted with a reflux condenser, dropping funnel, stopper and a magnetic stirrer. All exits were closed with drying tubes. The amide (XI) 40 g, 12.8 mmoles) in dry diethyl ether (1L) was added dropwise to a gently refluxing solution, over 1 hour. The condenser was transferred to the dropping funnel and the funnel was washed clean by reflux. The condenser was then returned to its former position and reflux maintained for 4 hours. Addition of water (5 ml) and magnesium sulphate heptahydrate (100 g) gave a solid which which was filtered and washed with water (2L) and diethyl ether in alternate small portions. Separation of the phases and extraction of the aqueous phase with ether (8 x 300 ml) followed by magnesium sulphate drying, filtration and evaporation gave a yellow oil, which was further dried by azeotrope with benzene. The aqueous phases had a pH of 11 which is a little too high for a phenol extraction. Accordingly, hydrochloric acid was added to pH5 and this was neutralised with sodium bicarbonate to pH8. However, further extraction only gave traces of 3-(4-hydroxyphenyl) propionic acid (confirmed by ¹H NMR).

TLC Product Rf 0.2 eluant 95% chloroform, 5% methanol ¹H NMR (CDC1₃) 7.4 ppm (5H, m, phenyl); 7.2 (2H, 8hz, aromatics, part of AB pair); 6.2 (1H, brs, OH); 2.5 (10H, 2H¹, 2H³, 2H⁴, 2H⁶, 2H⁷); 1.8 (4H m, 2H², 2H⁵); 1.0 (3H, t, 7hz, 3H⁸).

### Antispasmodic Activity

Antispasmodic activity was determined using standard methodology. (d)
Guinea-pig ileum muscle was suspended in a gut bath, in oxygenated Tyrode's solution at 37°. Doses of acetylcholine were added to the gut bath fluid, the contraction recorded, and the bath washed out with fresh Tyrode's solution. This was carried out repeatedly to establish a dose of acetylcholine to cause a sub-maximal response. This dose of acetylcholine was used repeatedly, on a 2 minute cycle to establish steady responses. Doses of antispasmodic were then added 30 seconds before the acetylcholine dose to establish the magnitude of reduction of the acetylcholine contraction.

Using atropine sulphate as a standard, N-ethyl, N-(3-phenyl propyl) 3-(4-hydroxyphenyl)-propylamine (II) in the hydrochloride form was shown to have an antispasmodic activity approximately 25 times less than the standard (1.5 ng of the said amine hydrochloride was equivalent to 0.06 ng of atropine sulphate.)

Literature references (a) - (d) are listed below.

### References

(a) T. Hudlicky, Reductions in Organic Chemistry, Ellis Horwood, Chichester, 1984.
(b) A.C. Cope and E Ciganek, Organic Synthesis, 1960, 39, 19.
(c) V. M. Micovic and M.L. Mihailovic, J. Org. Chem., 1953, 18, 1190.
(d) Pharmacological Experiments on Isolated Preparations, E & S Livingstone Edinburgh and London, 1970.

## Claims

1. A propylamine derivative having the general formula (I) 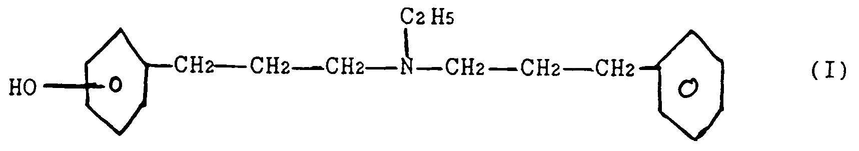 or a quaternary base thereof.

2. N-ethyl, N- (3- phenyl propyl), 3- (4-hydroxyphenyl) - propylamine or a quaternary base thereof.

3. A compound as defined in Claim 1 or Claim 2 which is in the form of a pharmaceutically acceptable inorganic salt.

4. A compound according to Claim 3 which is in the form of a hydrochloride thereof.

5. A compound as defined in Claim 1 or Claim 2 which is in the form of a pharmaceutically acceptable organic salt.

6. A compound according to Claim 5 which is in the form of a citrate thereof.

7. A composition comprising a compound as defined in any one of Claims 1 to 6 and a pharmaceutically acceptable carrier therefor.

8. A composition according to Claim 7 which is in the form of tablets, capsules, microspheres, liquid, semisolid or suppository dosage.

9. A method for the production of a compound (I) comprising coupling, using a coupling agent, a compound having the formula (III) 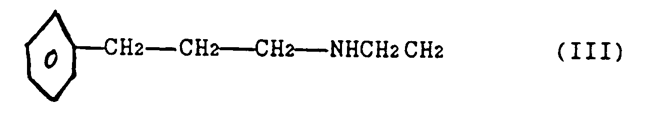 with a compound having the general formula (IV) 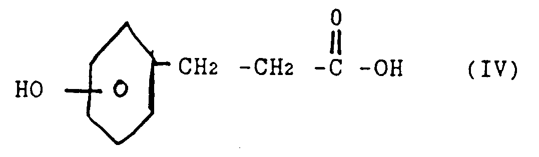 to produce a compound having the general formula (V) 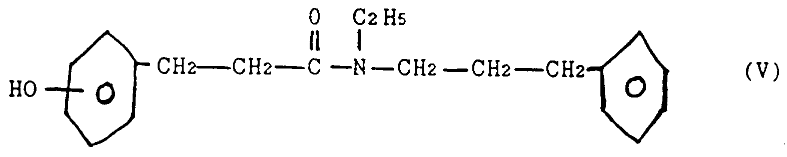 and reducing, using a reducing agent, the compound (V) to the amine having the general formula (I).

10. Method according to Claim 9, wherein the said coupling agent is dicyclohexyl carbodimide.

11. Method according to Claim 9 or Claim 10, wherein said reduction is achieved by the use of lithium aluminium hydride as reducing agent.

## Patentansprüche

1. Ein Propylaminderivat der allgemeinen Formel (I) 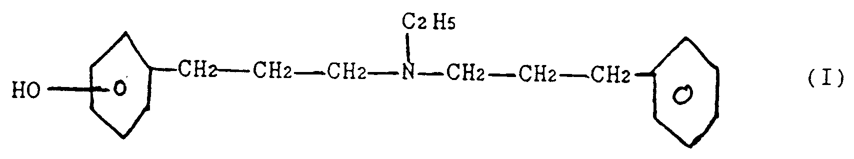 oder eine quartäre Base davon.

2. N-Äthyl, N-(3-Phenylpropyl), 3-(4-Hydroxyphenyl)-Propylamin oder eine quartäre Base davon.

3. Eine Verbindung nach Anspruch 1 oder Anspruch 2 in der Form eines pharmazeutisch annehmbaren anorganischen Salzes.

4. Eine Verbindung nach Anspruch 3 in der Form eines Hydrochlorids davon.

5. Eine Verbindung nach Anspruch 1 oder Anspruch 2 in der Form eines pharmazeutisch annehmbaren organischen Salzes.

6. Eine Verbindung nach Anspruch 5 in der Form eines Citrats davon.

7. Ein Präparat, das eine Verbindung nach einem der Ansprüche 1 bis 6 umfaßt, und ein pharmazeutisch annehmbarer Träger dafür.

8. Ein Präparat nach Anspruch 7 in der Form von Tabletten, Kapseln oder Mikrosphären bzw. in flüssiger, halbfester oder zäpfchenartiger Verabreichungsform.

9. Ein Verfahren zur Herstellung einer Verbindung (I), bei der eine Verbindung der Formel (III) 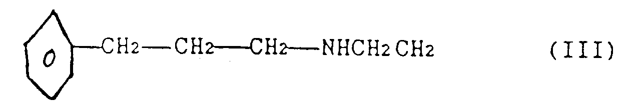 mit einer Verbindung der allgemeinen Formel (IV) 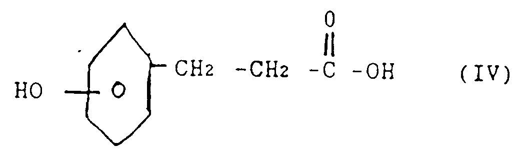 mit Hilfe eines Kupplungsmittels gekuppelt wird, um eine Verbindung der allgemeinen Formel (V) 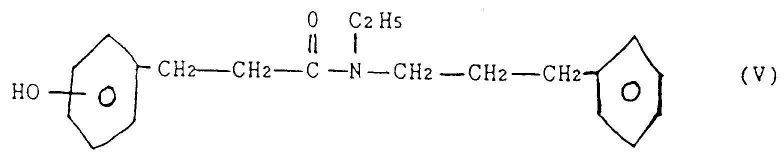 zu erzeugen, und bei der die Verbindung (V) mit Hilfe eines Reduktionsmittels auf das Amin der allgemeinen Formel (I) reduziert wird.

10. Verfahren nach Anspruch 9, bei dem das besagte Kupplungsmittel Dicyclohexylcarbodiimid ist.

11. Verfahren nach Anspruch 9 oder Anspruch 10, bei dem die besagte Reduktion mit Hilfe von Lithiumaluminiumhydrid als Reduktionsmittel erzielt wird.

## Revendications

1. Dérivé de propylamine ayant la formule générale (I) 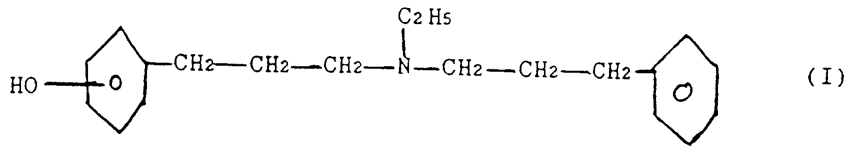 ou une base quaternaire de celui-ci.

2. N-éthyle, N-(3-phénylpropyle), 3-(4-hydroxyphényle)-propylamine ou une base quaternaire de celui-ci.

3. Composé selon la définition de la Revendication 1 ou de la Revendication 2 qui est sous la forme d'un sel inorganique acceptable pharmaceutiquement.

4. Composé selon Revendication 3 qui est sous la forme d'un hydrochlorure de celui-ci.

5. Composé selon la définition de la Revendication 1 ou de la Revendication 2 qui est sous la forme d'un sel organique acceptable pharmaceutiquement.

6. Composé selon Revendication 5 qui est sous la forme d'un citrate de celui-ci.

7. Composition comprenant un composé selon la définition de l'une quelconque des Revendications 1 à 6 et un support acceptable pharmaceutiquement pour celui-ci.

8. Composition selon Revendication 7 qui est sous forme de comprimés, de gélules, de microsphères, de liquide, de semi-solide ou de suppositoire.

9. Procédé pour la production d'un composé (I) comprenant le couplage, au moyen d'un agent de couplage, d'un composé ayant la formule (III) 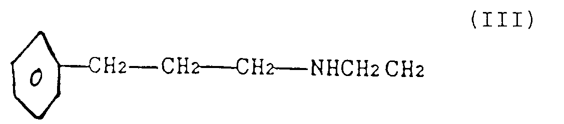 avec un composé ayant la formule générale (IV) 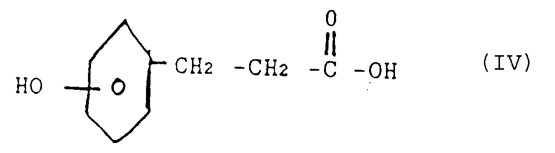 pour produire un composé ayant la formule générale (V) 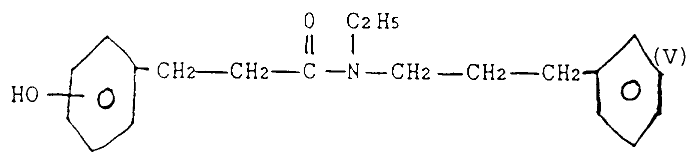 et la réduction, au moyen d'un agent de réduction, du composé (V) à l'amine ayant la formule générale (I).

10. Procédé selon Revendication 9, dans lequel ledit agent de couplage est du carbodiimide de dicyclohexyle.

11. Procédé selon Revendication 9 ou Revendication 10, dans lequel ladite réduction est réalisée par l'emploi d'hydrure d'aluminium-lithium comme agent de réduction.
